# EUROPEAN PATENT APPLICATION

(11) **EP 4 365 592 A1**
(43) Date of publication of application: **08.05.2024**
(21) Application number: 22842163.2
(22) Date of filing: 14.07.2022
(51) Int. Cl.: G01N 33/531, G01N 33/68, G01N 33/96

(54) **COMPOSITION CONTAINING TARC, DILUTED SOLUTION, METHOD FOR PREVENTING CARRY OVER OF TARC, ADSORPTION-PREVENTING AGENT, AND CONTINUOUS ANALYSIS METHOD**

(30) Priority: 15.07.2021 JP 2021116842
(71) Applicant: Sekisui Medical Co., Ltd., Tokyo 103-0027 (JP)
(72) Inventor: FUJIMURA, Kengo, Tokyo 103-0027 (JP); Sasaki, Ryuta, Tokyo 103-0027 (JP)
(74) Representative: Hoffmann Eitle
(86) International application number: PCT/JP2022/027642
(87) International publication number: WO 2023/286821

(57) **Abstract**

An object is to provide a diluent for a calibration sample and a sample that is less likely to cause a carryover of the TARC antigen. The object can be solved by a composition including (A) TARC (Thymus and activation-regulated chemokine) and (B) one or more components selected from the group consisting of acidic amino acids, basic amino acids, acidic polymers having an average molecular weight of 4,000 to 1,200,000, and salts thereof, and being in liquid form.

## Description

### Technical Field

The present invention relates to a composition containing TARC, and a diluent for a composition containing TARC. The present invention also relates to a method for reducing a TARC carryover, and an adsorption inhibitor for preventing adsorption of TARC. The present invention further relates to a method for continuously analyzing two or more samples containing TARC.

### Background Art

Thymus and activation-regulated chemokine (which may be hereinafter referred to as TARC) is C-C chemokine ligand 17 (CCL17) and is a kind of chemokine having a leukocyte migration activity. TARC attracts Th2 cells, which are one of lymphocytes, to the lesion site to cause IgE production and eosinophil infiltration/activation. It is considered that TARC exacerbates the symptoms of atopic dermatitis by enhancing allergic reactions in this way (Non Patent Literature 1).

Prompt and reliable sedation of atopic dermatitis inflammation is considered important. As compared with other indicators of the disease state of atopic dermatitis such as serum IgE level, peripheral blood eosinophil count, and serum LDH level, TARC matches well with the severity of atopic dermatitis and is considered to reflect the disease state more sensitively (Non Patent Literature 2). Accordingly, use of TARC as a biomarker enables the severity to be grasped objectively and quickly when selecting or changing therapeutic agents for atopic dermatitis, to determine the effects.

For measuring components in a sample, a calibration sample needs to be used. The calibration sample is a sample containing the component to be measured, which is used for applications such as an internal standard or a concentration calibration standard (calibrator). The calibration sample is preferably in the form of a fluid solution (which may be hereinafter referred to as "liquid") for ease of operation. In this case, examples of the matter desired for the calibration sample include maintenance of biological activity (such as antigenicity to specific antibodies, binding activity to specific binding partners of antibodies and lectins, physiological activity of peptide hormones, enzyme activity, and three-dimensional structure as a protein for supporting each activity), prevention of adsorption to containers, and maintenance of antiseptic ability.

As a method for storing a calibration sample in liquid form intended for use in an immunoassay, a method of stabilizing an antigen in coexistence with casein and/or whey protein in a calibration sample (Patent Literature 1), a method of stabilizing insulin in coexistence with a bile acid amide derivative (Patent Literature 2), and a method of stabilizing a soluble interleukin-2 receptor (sIL-2R) in coexistence with a chelating agent (Patent Literature 3) are known. For TARC, the present inventors further have studied on the method of using a cationic surfactant or an anionic surfactant (Patent Literature 4), a method of using one or more selected from the group consisting of sugar fatty acid ester-type nonionic surfactants, polyoxyethylene-polyoxypropylene block copolymer-type nonionic surfactants, and polyoxyethylene alkylamine-type nonionic surfactants (Patent Literature 5), and a method of using a polymer having 2-methacryloyloxyethyl phosphorylcholine as a structural unit (Patent Literature 6).

### Citation List

### Patent Literature

Patent Literature 1
   Japanese Patent Laid-Open No. 08-005634
Patent Literature 2
   Japanese Patent Laid-Open No. 08-012593
Patent Literature 3
   Japanese Patent Laid-Open No. 2010-230660
Patent Literature 4
   Japanese Patent Application No. 2020-119024
Patent Literature 5
   Japanese Patent Application No. 2020-119025
Patent Literature 6
   Japanese Patent Application No. 2020-119026

### Non Patent Literature

Non Patent Literature 1
   J Allergy Clin Immunol 107: 535-541, 2001
Non Patent Literature 2
   Journal of the Japanese Dermatological Association 116 (1): 27-39, 2006

### Summary of Invention

### Technical Problem

When measuring components in a sample using an autoanalyzer, the sample is dispensed into a dilution cell or a reaction cell using a sample probe. Specifically, a sample probe is a micro capillary tube for sucking up a required amount of a sample (a calibration sample, a sample, or a sample diluted with a diluent) and putting the sample into a reaction cell. The sample probe operates based on a particular program for every autoanalyzer. For concentration calibration, the sampling is carried out from the lower concentration side, and thus many apparatuses have a program for rinsing a sample probe with water for each sample, but not for washing with a detergent. Some apparatuses have a program for rinsing or washing with a detergent only at the end of completing samplings of all standard substances for calibration, and this case is conducted without rinsing a sample probe even with water for every sample. For this reason, a zero concentration or a low concentration calibration sample is sampled without washing of a sample probe, which has suctioned the high concentration standard substance for calibration when concentration calibration is carried out again because of an error and the like while sampling a high concentration calibration sample or after sampling and before rinsing or washing. The antigen contained in the high concentration calibration sample is likely to adhere to the sample probe and when not thoroughly rinsed with water, such a component contaminates the next sample, that is, a carryover occurs. Thus, the carryover occurred during concentration calibration fails to construct an accurate calibration curve, thereby posing a problem of failing to obtain an accurate measured value of a sample.

Additionally, during the sample measurement, a system generally involves rinsing a sample probe with water for each sample, and thus a carryover is likely to occur as with a calibration sample, however a carryover can be avoided by adding an apparatus setting (carryover avoidance setting) so that a sample probe is washed with a detergent before sampling. On the other hand, some autoanalyzer do not load the above washing mechanism, thereby posing a disadvantage of deteriorating the processing ability due to the sample probe washing. Solution to Problem

The present inventors found that when quantifying TARC continuously, the TARC antigen adsorbs onto a sample probe, thereby causing a carryover to a subsequent sample and thereafter to be quantified. The present inventors conducted extensive studies on a method in which no carryover occurs. As a result, the present inventors found that the occurrence of a carryover can be prevented when the following structure is employed, whereby the present invention has been accomplished.

Specifically, the present invention is as follows.
<1> A composition including:
   (A) TARC (Thymus and activation-regulated chemokine); and
   (B) one or more components selected from the group consisting of acidic amino acids, basic amino acids, acidic polymers having an average molecular weight of 4,000 to 1,200,000, and salts thereof, and
   being in liquid form.
<2> The composition according to <1>, wherein the composition is a calibration sample solution for measuring TARC.
<3> The composition according to <1> or <2>, further including:
   (C) a buffer.
<4> The composition according to any one of <1> to <3>, wherein the acidic polymer and a salt thereof are sodium polystyrene sulfonate, a naphthalene sulfonic acid condensate, sodium polyacrylate, or polyacrylic acid.
<5> The composition according to any one of <1> to <4>, wherein a concentration of the component (B) is 0.0001 to 20 mass% with respect to the composition.
<6> A diluent for a sample containing TARC, including:
   (B) one or more components selected from the group consisting of
   acidic amino acids, basic amino acids, acidic polymers having an average molecular weight of 4,000 to 1,200,000, and salts thereof.
<7> The diluent according to <6>, wherein the acidic polymer and a salt thereof are sodium polystyrene sulfonate, a naphthalene sulfonic acid condensate, sodium polyacrylate, or polyacrylic acid.
<8> The diluent according to <6> or <7>, further including:
   (C) a buffer.
<9> The diluent according to any one of <6> to <8>, wherein a concentration of the component (B) is 0.0001 to 20 mass% with respect to the diluent.
<10> A method for reducing a TARC carryover, including contacting:
   (A) TARC with
   (B) one or more components selected from the group consisting of acidic amino acids, basic amino acids, acidic polymers having an average molecular weight of 4,000 to 1,200,000, and salts thereof in a solution.
<11> The method for reducing a TARC carryover according to <10>, wherein the acidic polymer and a salt thereof are sodium polystyrene sulfonate, a naphthalene sulfonic acid condensate, sodium polyacrylate, or polyacrylic acid.
<12> The method for reducing a TARC carryover according to <10> or <11>, wherein the solution is a buffer.
<13> The method for reducing a TARC carryover according to any one of <10> to <12>, wherein the carryover is a carryover in a sample probe.
<14> The method for reducing a TARC carryover according to any one of <10> to <13>, wherein a concentration of the component (B) is 0.0001 to 20 mass% with respect to the solution.
<15> An adsorption inhibitor for preventing adsorption of TARC in a solution containing TARC onto a sample probe, including:
   (B) one or more components selected from the group consisting of acidic amino acids, basic amino acids, acidic polymers having an average molecular weight of 4,000 to 1,200,000, and salts thereof.
<16> The adsorption inhibitor according to <15>, wherein the acidic polymer and a salt thereof are sodium polystyrene sulfonate, a naphthalene sulfonic acid condensate, sodium polyacrylate, or polyacrylic acid.
<17> A method for continuously analyzing two or more samples containing TARC, the method including:
   (a) suctioning a first sample containing TARC using a sample probe,
   (b) discharging the first sample from the sample probe,
   (c) measuring a signal intensity of the first sample,
   (d) rinsing the wall surface of the sample probe with a rinsing solution,
   (e) suctioning a second sample containing TARC using the sample probe, and
   (f) measuring a signal intensity of the second sample,

   wherein the first sample and the second sample include (B) one or more components selected from the group consisting of acidic amino acids, basic amino acids, acidic polymers having an average molecular weight of 4,000 to 1,200,000, and salts thereof, at 0.0001 mass% to 20 mass%, respectively, with respect to the first sample and the second sample, and
   a TARC concentration of the first sample containing TARC is higher than a TARC concentration of the second sample containing TARC.

### Advantageous Effects of Invention

The present invention can provide a diluent for a calibration sample and a sample that is less likely to cause a carryover of the TARC antigen. Accordingly, the present invention enables TARC in a biological sample to be accurately quantified and the severity of atopic dermatitis to be accurately grasped.

### Description of Embodiments

Hereinafter, the present invention will be described in detail. The present description is described in divided aspects of the present invention, but sections, definitions of terms, and embodiments described in respective aspects are also applicable to other aspects.

### 1. Composition containing TARC

### (Component (A): TARC)

In this description, "TARC" means Thymus and activation-regulated chemokine (CCL17). TARC is a kind of chemokine having leukocyte migration activity. TARC has the function to attract Th2 cells, which are one of lymphocytes, to the lesion site to cause IgE production and eosinophil infiltration/activation. Use of TARC as a biomarker enables the severity to be grasped objectively and quickly when selecting or changing therapeutic agents for atopic dermatitis.

TARC can be measured by a known method such as immunological technique. Examples of the immunological technique include ELISA, enzyme immunoassay, surface plasmon resonance, latex agglutination immunoassay (LTIA, Latex turbidimetric immunoassay), chemiluminescence immunoassay, electrochemiluminescence immunoassay, fluorescent antibody method, radioimmunoassay, Western blotting, immunochromatography, and high-performance liquid chromatography (HPLC).

The TARC to be contained in the composition of the present invention may be a commercially available product or one produced or purified by oneself. The TARC to be contained in the composition of the present invention may be produced in vitro or extracted from the living body.

### (Concentration of TARC)

The concentration of the TARC to be contained in the composition of the present invention is not limited to the following examples but is preferably 10 pg/mL to 1 µg/mL, more preferably 50 pg/mL to 500 ng/mL, further preferably 100 pg/mL to 100 ng/mL, and most preferably 100 pg/mL to 50 ng/mL, with respect to the composition.

### (Component (B))

The composition of the present invention includes one or more components selected from the group consisting of acidic amino acids, basic amino acids, acidic polymers having an average molecular weight of 4,000 to 1,200,000, and salts thereof (hereinafter, simply referred to as component (B)). These components are ionized in an aqueous solution and show a positive charge or a negative charge. Being ionized in an aqueous solution and showing a negative charge refers to having many more acidic functional groups such as a sulfo group than basic functional groups such as an amino group, and showing a negative charge when dissolved in water. Being ionized in an aqueous solution and showing a positive charge refers to having many more basic functional groups than acidic functional groups, and showing a positive charge when dissolved in water.

Examples of the acidic functional group include sulfo groups, phospho groups, and carboxyl groups. Sulfo groups or carboxyl groups are preferable.

Examples of the basic functional group include amino groups such as primary amino groups, secondary amino groups, tertiary amino groups, quaternary ammonium salts, amide groups, pyridyl groups, pyridine groups, pyrrolidone groups, imidazole groups, and imine groups. Amino groups are preferable.

For the acidic amino acids, acidic polymers having a weight average molecular weight of 4,000 to 1,200,000, or salts thereof, L-glutamic acid, L-aspartic acid, sodium polystyrene sulfonate (CAS Registered Number 25704-18-1), sodium polyacrylate (CAS Registered Number 9003-04-7), naphthalene sulfonic acid condensate (CAS Registered Number 9084-06-4), or polyacrylic acid (CAS Registered Number 9003-01-4) can be used. The naphthalene sulfonic acid condensate usable is produced and sold by Kao Corporation as DEMOL (registered trademark) RN. The amino acid stated in this description can be α-amino acid to be a structural unit of the living body, or can be a non-natural amino acid. In this description, the amino acids include L-form, D-form, and mixtures thereof.

### (Basic amino acids or salts thereof)

For the basic amino acids, or salts thereof, L-lysine, L- arginine, or salts thereof can be used.

The salts of acidic amino acids, the salts of basic amino acids, or the salts of acidic polymers having a weight average molecular weight of 4,000 to 1,200,000 are not limited as long as the effects of the present invention can be obtained, and examples include salts formed with an inorganic acid or an organic acid such as hydrochloride, hydrosulfate, and acetate, and salts formed with a base such as sodium salt, and potassium salt.

The acidic amino acids, basic amino acids, or acidic polymers having a weight average molecular weight of 4,000 to 1,200,000 are preferably L-glutamic acid, L-aspartic acid, sodium polystyrene sulfonate, naphthalene sulfonic acid condensate, or polyacrylic acid, and more preferably L-glutamic acid, L-aspartic acid, sodium polystyrene sulfonate, or naphthalene sulfonic acid condensate (DEMOL RN). The acidic polymers usable are those having an average molecular weight (Average MW: weight average molecular weight) of, for example 4,000 to 1,200,000, and preferably 5,000 to 1,000,000. Further, the weight average molecular weight (MW) is preferably 70,000 to 1,000,000. The weight average molecular weight (MW) can be measured using size exclusion chromatography (SEC). The acidic polymers can be used without limitation as long as it is a polymer obtained by polymerizing a monomer having an acidic group. The acidic polymer is preferably an acidic polymer obtained by polymerizing a monomer having a carboxyl group or a sulfo group.

The concentration of component (B) is, for example, 0.0001 to 20 mass%, and preferably 0.001 to 10 mass%, with respect to the composition.

When an acidic amino acid or a salt thereof is used, the concentration is more preferably 0.05 to 10 mass%, further preferably 0.1 to 5 mass%, and most preferably 0.5 to 1 mass%, with respect to the composition.

When an acidic polymer or a salt thereof is used, the concentration is more preferably 0.001 to 1 mass%, further preferably 0.001 to 0.5 mass%, and most preferably 0.002 to 0.1 mass%, with respect to the composition.

When a basic amino acid or a salt thereof is used, the concentration is, for example, 1 to 20 mass%, preferably 3 to 18 mass%, more preferably 5 to 15 mass%, and most preferably 5 to 10 mass%, with respect to the composition.

In the composition of the present embodiment, the order of adding component (B) and TARC is not particularly limited as long as the effects of the present invention can be obtained.

### (Component (C): buffer)

In this description, the "buffer" means a solution having a pH buffering action.

For the buffer used in the present invention, known buffers can be appropriately used such as PBS (phosphate buffered saline), MES (2-(N-morpholino)ethanesulfonic acid), PIPES (piperazine-N,N'-bis(2-ethanesulfonic acid), ACES (N-(2-Acetamido)-2-aminoethanesulfonic acid), ADA (N-(2-Acetamido)iminodiacetic acid), Bis-Tris (2,2-Bis(hydroxyethyl)-(iminotris)-(hydroxymethyl)-methane), Tris (tris(hydroxymethyl)aminomethane), MOPS (3-Morpholinopropanesulfonic acid), HEPES (4-(2-hydroxyethyl)-1-piperazineethanesulfonic acid), citrate buffer, glycine buffer, borate buffer, or phosphate buffer, but not limited thereto. The buffer used in the present invention is preferably PBS.

The concentration of the buffer is not particularly limited as long as the effects of the present invention can be obtained and, for example, 1 to 500 mM, 5 to 400 mM, 10 to 300 mM, or 50 to 200 mM.

The buffer pH used in the present invention is, for example, 4.0 to 11.0, 5.0 to 10.0, 6.0 to 9.0, 6.5 to 8.0, or 7.0 to 8.0. The pH can be adjusted using a reagent for pH adjustment known to those skilled in the art such as sodium hydroxide or hydrochloric acid.

The buffer content in the composition of the present invention is, for example, 90 mass% or more, 92 mass% or more, 95 mass% or more, 97 mass% or more, or 99 mass% or more, with respect to the composition.

### (Storage container)

The composition of the present invention is preferably filled in a storage container. The material of the storage container is not specifically limited, as long as the effects of the present invention can be obtained, and sealing can be achieved, but at least part or all of the contact area with the composition is plastic [such as an olefinic resin, a styrenic resin, an acrylic resin, a polyester resin, a polycarbonate resin, a fluorine resin, a chlorinated resin (such as polyvinyl chloride), a polyamide resin, a polyacetal resin, a polyphenylene ether resin (such as modified polyphenylene ether), polyarylate, polystyrene sulfone, a polyimide resin, a cellulose resin (such as cellulose acetate), and a hydrocarbon resin (including a halogen -substituted product)], a metal (such as aluminum), glass, or the like. Among them, in view of production, transportation, and storage of a calibration sample, plastic or glass is preferable, an olefin resin is preferable among plastics, and polypropylene is more preferable.

The storage container may be made of a single material or two or more materials but is preferably made of a single material. The storage container can include a container body and a cap. In this case, the container body and the cap may be made of different materials. In addition, the storage container, particularly the body part, preferably has transparency to the extent that the liquid content can be seen from the outside.

The form of the storage container may be either hard type or soft type, and examples thereof include ampoules, vials, soft bags, syringe-type containers, and glass bottles. The storage container is preferably in the form of a plastic eyedropper bottle, particularly in the form of a cylindrical eyedropper bottle, including a container body and a cap, for the ease of use and the stability of

### TARC.

### (Composition)

The composition of the present invention can be used as a calibration sample solution in the measurement of TARC. In this description, the calibration sample solution means a sample solution that is used for accurately measuring a substance to be measured and contains the substance to be measured at a certain concentration. Examples thereof include a standard substance, a calibrator, a control, and an internal standard substance. Examples of supply forms of the composition of the present invention include a solution state prepared in advance by mixing of a solvent with TARC and component (B).

In the TARC measurement, "using a composition" means using a composition to measure TARC accurately. For example, it means using a liquid composition containing TARC as a calibration sample solution (such as a standard substance, a calibrator, a control, and an internal standard substance).

The pH of the composition of the present invention is, for example, 4.0 to 11.0, 5.0 to 10.0, 6.0 to 9.0, 6.5 to 8.0, or 7.0 to 8.0.

The pH can be adjusted by using a pH adjusting reagent well known to those skilled in the art, such as sodium hydroxide or hydrochloric acid.

The composition of the present invention may have any composition that is not particularly limited as long as it does not impair the effects of the present invention. When TARC is measured by immunoassay, the composition is suitable as long as it does not impair the effects of the present invention, and does not prevent all or part of the reactions constituting the assay system such as antigen-antibody reaction, and enzymatic reaction. Various components generally used in the immunoassay can be appropriately selected and used according to the purpose, including, for example, components that promote antigen-antibody reactions (e.g., polymers such as polyethylene glycol and polyvinylpyrrolidone), glycoproteins and peptides (e.g., BSA and casein), amino acids, salts (e.g., sodium chloride and potassium chloride), saccharides (e.g., sucrose and cyclodextrin), preservatives (e.g., sodium azide and ProClin 300), and other adsorption inhibitors (e.g., Lipidure).

In this description, the "carryover" means that when a first sample is separated, TARC antigens in the sample adsorb and reside onto a wall surface of a sample probe and the like, and the TARC antigens derived from the first sample flow into a reaction cell of the second sample and thereafter to be subsequently sampled. When a carryover occurs, signal intensities of the second sample and thereafter are comparatively higher than the signal intensities originally obtained, thereby failing to obtain an accurate quantitative value. The present inventors found, as described earlier, that when TARC contacts with a sample probe even only for a short time, a carryover occurs. When the composition of the present invention is used, a carryover can be prevented, and the accuracy of the TARC measurement can be improved.

In this description, the sample probe means a micro capillary tube for sucking up a required amount of a sample from a container such as a blood drawing tube and putting the sample into a reaction cell. The sample probe includes both of those for automatic measuring apparatus and those for using manually. The sample probe is preferably made of a metal, and more preferably made of a stainless steel.

### (Sample for measurement of TARC)

The sample for TARC measurement is not particularly limited as long as TARC can be measured, but blood, serum, or blood plasma is preferably used. The sample may be appropriately pretreated, as required. The sample is preferably a biological sample collected from a human.

### (Reagent kit for TARC)

The composition of the present invention can be provided in the form of a reagent kit for TARC including the composition of the present invention and other reagents. Example of the reagent kit include a reagent kit using an immunological technique. Examples of the immunological technique include ELISA, enzyme immunoassay, surface plasmon resonance, latex agglutination immunoassay (LTIA, Latex turbidimetric immunoassay), chemiluminescence immunoassay, electrochemiluminescence immunoassay, fluorescence antibody method, radioimmunoassay, Western blotting, immunochromatography, and high-performance liquid chromatography (HPLC).

The reagent kit can be used for selecting a treatment method or drug for atopic dermatitis, and for grasping the severity of atopic dermatitis when determining the effects of treatment.

The reagent kit can also include instructions for use and the like. The reagent kit may contain optional components such as a stabilizer, a pH adjuster, and a reaction container.

### 2. Diluent for a sample containing TARC

### (Sample containing TARC)

The diluent of the present invention can be used for diluting a sample containing TARC. Examples of the sample containing TARC include samples for measuring TARC (e.g., blood, serum, or blood plasma), standard substances, calibrators, controls, and internal standard substances. Other compositions excluding TARC are the same as the "1. Composition containing TARC" described earlier.

The diluent of the present invention can be used so that the TARC concentration after dilution is preferably 10 pg/mL to 1 µg/mL, more preferably 50 pg/mL to 500 ng/mL, further preferably 100 pg/mL to 100 ng/mL, and most preferably 100 pg/mL to 50 ng/mL.

### 3. Method for reducing a TARC carryover

The method for reducing a TARC carryover of the present invention includes contacting TARC with component (B) in a solution. TARC may be added after component (B) is added to a solution, or component (B) may be added after TARC is added to a solution. The solution is preferably a buffer, and more preferably PBS.

The carryover include a carryover caused by a sample probe. As long as the effects of the present invention can be obtained, TARC to be component (A) and the compound found in the present application to be component (B) may be contacted with each other in a sample probe, or the composition containing TARC prepared after contacting component (A) with component (B) may be transferred to a sample probe.

The method for reducing a TARC carryover of the present invention can include holding the prepared composition containing TARC in a sample probe. The "holding the prepared composition containing TARC in a sample probe" refers that the composition containing TARC may only be in contact with the wall surface of a sample probe, and other movements such as moving the sample probe for dispensing can be simultaneously carried out.

The holding time of TARC in a sample probe is not limited as long as the effects of the present invention can be obtained and, for example, the lower limit can be 0.1 sec or more, 0.2 sec or more, or 0.5 sec or more. The upper limit can be 1 hour or less, 30 min or less, 15 min or less, 10 min or less, 5 min or less, 1 min or less, 30 sec or less, 10 sec or less, 5 sec or less, 3 sec or less, or 2 sec or less. Specific holding time can range from 0.1 sec to 1 hour, 0.1 sec to 30 min, 0.1 sec to 15 min, 0.1 sec to 10 min, 0.1 sec to 1 min, 0.2 sec to 30 sec, 0.2 sec to 10 sec, 0.5 sec to 5 sec, or 0.5 sec to 3 sec.

The method for reducing a TARC carryover of the present invention can further include, optionally, the following one or more steps:
(a) suctioning a first sample containing TARC using a sample probe,
(b) discharging the first sample from the sample probe,
(c) measuring a signal intensity of the first sample,
(d) rinsing the wall surface of the sample probe with a rinsing solution,
(e) suctioning a second sample containing TARC (and the samples thereafter) using the sample probe,
(f) discharging the second sample (and the samples thereafter) from the sample probe,
(g) measuring a signal intensity of the second sample and the samples thereafter.

In step (a), the first sample containing TARC and the second sample containing TARC and the samples thereafter all include component (B). Component (B) contained in the first sample and component (B) contained in the second sample are preferably the same. The first sample and the second sample can have component (B) added thereto as a sample diluent before measurement, or can also include component (B) from the beginning. The first sample and the second sample can be any of samples for measuring TARC (e.g., blood, serum, or blood plasma), or calibration samples (e.g., standard substances, calibrators, controls). The first sample and the second sample are preferably calibration samples for measuring TARC (e.g., standard substances, calibrators, controls).

The "suctioning using a sample probe" in steps (a) and (e) includes the suction of the first sample manually by an analyzer, and the suction of the first sample automatically using an autoanalyzer when an analyzer turns on a switch. The same also applies to steps other than (a) The autoanalyzer usable includes autoanalyzer for carrying out latex immunonephelometry (LTIA method, Latex immunoturbidimetric assay), electrochemiluminescence immunoassay (ECL method), enzyme immunoassay (ELISA), chemiluminescence immunoassay, fluorescent antibody method, and the like.

In steps (a) to (g), steps (a), (b), (d), (e), and (f) are carried out in the order listed. The timing for carrying out (c) and (g) can be set as desired according to the program of an autoanalyzer to be used.

The substance for generating a signal can be appropriately selected according to the kind of analysis method. Examples of the substance for generating a signal include metal complexes, enzymes, insoluble particles, fluorescent substances, chemiluminescent substances, biotin, avidin, radioisotopes, gold colloidal particles, latex particles, and colored latexes. The signal is not limited as long as a TARC concentration in a sample can be measured based on an intensity of the signal, and can be appropriately selected from fluorescence, absorbance, scattered light intensity or the like according to the kind of analysis method.

In step (b), when the first sample does not include component (B), the TARC antigen has adhered to the wall surface of the sample probe after discharging the first sample. Accordingly, when a washing step using a detergent or the like is not provided after discharging the first sample, a carryover of the TARC antigen occurs to the second sample and the samples thereafter to be subsequently measured.

In step (d), the "rinsing the wall surface of the sample probe" means to flush the wall surface of the sample probe with a friction force, a shear stress, and the like, generated when a rinsing solution runs, without utilizing the actions such as rubbing and polishing with a brush, and the like. The rinsing solution is often substantially made only of water and more preferably substantially free of a detergent. This is because, when a rinsing solution includes a detergent, the detergent remained in a sample probe needs to be further rinsed off with water, thereby extending the time required for the rinsing step. It does not necessarily exclude that the rinsing solution includes a detergent, but the content of a detergent in the rinsing solution is preferably 0.1 mass% or less, more preferably 0.01 mass% or less, further preferably 0.001 mass% or less, and most preferably free of a detergent.

The detergent means a substance capable of detaching proteins from the wall surface of a sample probe based on a chemical action. The detergent specifically includes acid, alkali, a surfactant, an enzyme, and the like.

In step (e), the TARC concentration of the first sample containing TARC is preferably higher than the TARC concentration of the second sample and the samples thereafter containing TARC. When the TARC concentration of the first sample containing TARC is higher than the TARC concentration of the second sample containing TARC, signal intensities of the second sample and the samples thereafter are comparatively higher than signal intensities originally obtained due to the impact by the carryover of the TARC antigen, thereby likely failing to obtain an accurate quantitative value.

The TARC concentration of the first sample immediately before being discharged from a sample probe is, for example, 500 pg/mL or more, 1,000 pg/mL or more, 5,000 pg/mL or more, 10,000 pg/mL or more, 15,000 pg/mL or more, or 20,000 pg/mL or more.

The TARC concentration of the second sample immediately before being discharged from a sample probe may be lower than the TARC concentration of the first sample, and is, for example, 0 pg/mL to 10,000 pg/mL, 0 pg/mL to 5,000 pg/mL, 0 pg/mL to 1,000 pg/mL, 0 pg/mL to 700 pg/mL, 0 pg/mL to 600 pg/mL, 0 pg/mL to 500 pg/mL, 0 pg/mL to 300 pg/mL, or 0 pg/mL to 100 pg/mL. The above concentrations, when the sample diluent is added, are the concentrations after dilution.

In step (g), when the first sample includes component (B), the impact by the carryover of the TARC antigen to the second sample and the samples thereafter can be prevented. Accordingly, the TARC concentration can be measured with good accuracy.

In the method for reducing a carryover of the TARC antigen of the present invention can further include, optionally, the following one or more steps:
- calculating TARC concentrations of the first sample and the second sample and the samples thereafter, respectively, based on the signal intensities of the first sample and the second sample and the samples thereafter
- constructing calibration curves based on the signal intensities of the samples containing the first sample and the second sample
- washing the sample probe using a washing solution containing a detergent at the beginning of the sample analysis and/or at the completion of the sample analysis.

The "at the completion of the sample analysis" means the time when the analyses of all the samples to be analyzed is completed.

### 4. Adsorption inhibitor for preventing adsorption of TARC antigen

The adsorption inhibitor for preventing adsorption of TARC antigen of the present invention includes, as active component, a compound showing a positive or a negative charge. The embodiment of the adsorption inhibitor for preventing adsorption of TARC antigen of the present invention is the same as the embodiments described earlier.

### 5. Method for continuously analyzing two or more samples containing TARC

The "method for continuously analyzing two or more samples" in this description means to continuously analyze two or more samples using a fully autoanalyzer.

In the method for continuously analyzing two or more samples containing the TARC antigen of the present invention, it is preferable to analyze two or more samples without including a washing step using a detergent and the like between analyses of each sample. That is, in the method for continuously analyzing two or more samples containing the TARC antigen of the present invention, it is preferable to rinse the wall surface of a sample probe with water between analyses of each sample, and include a washing step using a detergent and the like at the beginning and completion of the analysis. In the method for continuously analyzing two or more samples, it does not necessarily exclude the system of washing the wall surface of a sample probe with a detergent between analyses of each sample.

The method for continuously analyzing two or more samples containing the TARC antigen of the present invention can include, optionally, contacting TARC to be component (A) with component (B) in a solution. The step can be a step of, for example, diluting the first and/or the second sample and the samples thereafter containing TARC with a sample diluent containing component (B).

The present invention will be specifically described by way of Examples, but these do not limit the scope of the present invention. In this description, % refers to mass%, unless otherwise specified. The product names, ingredient names, and distributors of the additives used in the examples are as follows.
- LIPIDURE (R) Series, available from NOF CORPORATION
- ACETAMIN (R) 24 ingredient name: Coconut amine acetates, available from Kao Corporation
- DEMOL (R) RN-L ingredient name: sodium salt of β-naphthalene sulfonic acid condensate, available from Kao Corporation
- ENAGICOL (R) L-30AN ingredient name: sodium lauroyl methyl β-alanine, available from Lion Corporation
- BlockMaster (R) CE series available from MBL Life Science

Other general reagents were purchased appropriately from reagent manufacturers and used.

### Examples

### 1. Measurement method

### 1-1. Measuring reagent

A LTIA measuring reagent for TARC measurement composed of the following first reagent and second reagent was prepared.

### (1) First reagent

100 mM MOPS-NaOH (PH 7.5)
500 mM NaCl
0.5% BSA

### (2) Second reagent

### Anti-human TARC monoclonal antibody sensitized latex (two types)

5 mM MOPS-NaOH (PH 7.0)

Anti-Human TARC monoclonal antibodies were obtained using commercially available TARC antigens by methods well known to those skilled in the art. Examples of the commercially available TARC antigens included CCL17, thymus and activation regulated chemokine (Shenandoah Biotechnology, Inc.), CCL17/TARC, Human (LifeSpan Bioscience, Inc.), and Human TARC (CCL17) (Abeomics, Inc.). Further, a combination of monoclonal antibodies capable of a sandwich assay against the TARC antigen was selected by a method well known to those skilled in the art. The anti-human TARC monoclonal antibody sensitized latex was prepared with reference to the method described in Japanese Patent Laid-Open No. 2017-181377.

### 1-2. Sample

The TARC antigen was dissolved in a diluent having the following composition to prepare a low concentration sample (sample A, 800 pg/mL level) and a high concentration sample (sample B, 20,000 pg/mL level). Additionally, saline was separately prepared.

### Diluent

- PBS (pH 7.2)
- 0.5% Lipidure (provided that this is different from the ingredient shown in Table 5)
- Additives (see Tables 2 to 6 for concentrations and ingredient names)

### 1-3. Measurement procedure

Combining the first reagent and the second reagent, TARC concentrations of the samples were measured using a HITACHI autoanalyzer 3500 (the sample probe was made of stainless steel). Specifically, 120 µL of the first reagent was added to 2.4 µL of the sample and heated at 37°C for 5 minutes, and subsequently 40 µL of the second reagent was added and stirred. Thereafter, absorbance changes with agglomeration formation for 5 minutes were measured at a dominant wavelength of 570 nm and a secondary wavelength of 800 nm. The measurement sensitivity was calculated from the obtained absorbance changes.

### 1-4. Analysis method

With the respective diluents in which each additive was used, the total of 12 samplings including twice of saline, five times of sample A (= first set), twice of sample B, five times of sample A (= second set) was carried out after rinsing the sample probe with water, but without washing using a detergent, and continuously measured. From the measurement sensitivity of each measurement, the average measurement sensitivity of two saline measurements was subtracted. The average measurement sensitivity of the five measurements at the first set was calculated, and the relative ratio (%) of each measurement sensitivity at the second set to the average measurement sensitivity at the first set was calculated. The holding time in the sample probe per measurement was about 1 to 2 seconds.

### 2. Results

Table 1 shows the results obtained when component (B) was not added to the calibrator diluent. The measurement sensitivities of the five measurements at the first set of sample A (800 pg/mL level) ranged from 20.9 to 22.1 mAbs, and the average measurement sensitivity was 21.4 mAbs. Subsequently, sample B (20,000 pg/mL level) was measured twice, and then sample A was measured again five times (second set). The measurement sensitivity of the first time at second set was 38.3 mAbs, the value was significantly higher than the average measurement sensitivity at the first set, and the relative ratio was as poor as 178.5%. The cause was presumably considered that the TARC antigen contained in sample B adsorbed onto the sample probe, and a carryover brought into the measurements at the second set occurred. The measurement sensitivities decreased as the measurements repeated second time, and third time, at the second set, but the relative ratio was 120.2 (%) even at the fifth time, indicating the impact of the carryover.

**[Table 1]**

| | | Comparative Example 1 | | |
|---|---|---|---|---|
| | | Measurement sensitivity (mAbs) | Average Measurement sensitivity (mAbs) | Relative ratio (%) |
| 1 set | First time | 20.9 | 21.4 | - |
| | Second time | 21.1 | | - |
| Sample A | | | | |
| Low concentration sample (n=5) | Third time | 21.7 | | - |
| | Fourth time | 21.4 | | - |
| | Fifth time | 22.1 | | - |
| Sample B | First time | 275.7 | - | - |
| High concentration sample (n=2) | Second time | 276.5 | | - |
| 2 set | First time | 38.3 | - | 178.5 |
| | Second time | 32.4 | | 151.4 |
| Sample A | | | | |
| Low concentration sample (n=5) | Third time | 30.3 | | 141.2 |
| | Fourth time | 29.0 | | 135.1 |
| | Fifth time | 25.8 | | 120.2 |

Then, Table 2 to Table 6 show the results obtained when various additives were added to the diluent so that the final concentrations shown in the tables were achieved. In the tables, relative ratios of each measurement sensitivity at the second set to the average measurement sensitivity of the five measurements at the first set were excerpted and shown.

**[Table 2]**

| Results when adding acidic amino acid | | | | | | | |
|---|---|---|---|---|---|---|---|
| | | Relative ratio (%) | | | | | Determination |
| | | First time | Second time | Third time | Fourth time | Fifth time | |
| Comparative Example 1 | Not added (Control) | 178.5 | 151.4 | 141.2 | 135.1 | 120.2 | - |
| Example 1 | + 1.0% Glutamic acid | 102.6 | 103.2 | 101.9 | 96.8 | 100.7 | A |
| Example 2 | + 0.5% Glutamic acid | 108.1 | 99.2 | 101.5 | 99.0 | 98.8 | A |
| Example 3 | + 0.5% Aspartic acid | 104.2 | 97.8 | 101.4 | 99.6 | 101.6 | A |

**[Table 3]**

| Results when adding basic amino acid, and neutral amino acid | | | | | | | |
|---|---|---|---|---|---|---|---|
| | | Relative ratio (%) | | | | | Determination |
| | | First time | Second time | Third time | Fourth time | Fifth time | |
| Comparative Example 1 | Not added (Control) | 178.5 | 151.4 | 141 2 | 135.1 | 120.2 | - |
| Example 4 | + 5.0% Arginine | 132.2 | 110.0 | 100.7 | 103.8 | 105.5 | B |
| Example 5 | + 10% Arginine | 117.9 | 99.5 | 96.5 | 98.6 | 88.5 | B |
| Example 6 | + 5.0% Lysine | 140.4 | 104.7 | 97.7 | 100.6 | 98.1 | B |
| Example 7 | + 10% Lysine | 126.0 | 98.2 | 103.3 | 98.0 | 102.5 | B |
| Comparative Example 2 | + 1.0% Histidine | 218.7 | 176.4 | 162.1 | 149.8 | 144.0 | D |
| Comparative Example 3 | + 1.0% Glycine | 179.7 | 159.6 | 146.6 | 140.8 | 133.4 | D |
| Comparative Example 4 | + 5.0% Glycine | 182.7 | 161.7 | 131.3 | 138.5 | 131.8 | D |
| Comparative Example 5 | + 10% Glycine | 176.1 | 153.1 | 146.5 | 133.8 | 130.0 | D |
| Comparative Example 6 | + 1.0% Methionine | 125.4 | 118.8 | 116.1 | 113.9 | 106.5 | C |
| Comparative Example 7 | + 10% Proline | 129.3 | 120.5 | 118.6 | 116.7 | 113.1 | C |
| Comparative Example 8 | + 1.0% Proline | 123.2 | 115.7 | 114.0 | 111.4 | 111.4 | C |
| Comparative Example 9 | + 1.0% Threonine | 126.1 | 115.8 | 118.2 | 111.9 | 110.7 | C |
| Comparative Example 10 | + 1.0% Phenylalanine | 129.6 | 118.5 | 119.6 | 119.0 | 114.6 | C |
| Comparative Example 11 | + 1.0% Asparagine. monohydrate | 123.3 | 116.4 | 116.2 | 110.9 | 115.0 | C |
| Comparative Example 12 | + 1.0% Glutamine | 124.3 | 120.7 | 117.9 | 115.0 | 111.4 | C |

First, Table 2 and Table 3 are described. Table 2 shows the results obtained when an acidic amino acid was added, and Table 3 shows the results obtained when a basic amino acid or a neutral amino acid was added. Under the "Determination" column, "A" shows that the relative ratio of the first time at the second set was within 90 to 110%, thereby being a very good result, "B" shows that the relative ratio of the first time at the second set deviated from 90 to 110%, but the relative ratio of the second time was within 90 to 110%, thereby being a good result, "C" shows that the relative ratios of the first and second times at the second set deviated from 90 to 110% but the third time was 80 to 120%, thereby being insufficient, while being effective when compared with not added, and "D" shows that no effect was found.

Table 2 reveals that when 0.5% to 1.0% of glutamic acid, which is an acidic amino acid, was added (Examples 1, 2), the relative ratio of the measurement sensitivity of the first time at the second set to the average measurement sensitivity of the five measurements at first set was 102.6 to 108.1%, reducing the carryover of sample B. When 0.5% of aspartic acid, which is an acidic amino acid, was added, the relative ratio of the measurement sensitivity of the first time at the second set to the average measurement sensitivity of the five measurements at first set was 104.2%, reducing the carryover as in the glutamic acid (Example 3).

As further shown in Table 3, when arginine, which is a basic amino acid, was added at high concentrations (5.0%, 10%), the tendency of reducing the carryover was found (Examples 4, 5). As in arginine, when lysine, which is a basic amino acid, was added at high concentrations, the tendency of reducing the carryover was found (Examples 6, 7). On the other hand, when a neutral amino acid was added, the reduction effect on carryover as found with the acidic amino acids was not found (Comparative Examples 2 to 12).

The above findings revealed that the diluent, when an acidic amino acid was added thereto, was capable of reducing the carryover caused by the TARC antigen, even at a concentration as low as about 0.5% to 1.0%, adsorbed onto the sample probe. Accordingly, it was considered that when an acidic amino acid was added, the measurement of a sample having a low concentration of the TARC antigen is possible with high accuracy even when the sample having a low concentration of the TARC antigen is measured immediately after a sample having a high concentration of the TARC antigen is measured.

Similarly, it was revealed that a basic amino acid, when added to the diluent at concentrations as high as 5.0% to 10%, had the reduction effect on the carryover caused when the TARC antigen adsorbs onto the sample probe.

**[Table 4]**

| Results when adding acidic polymer, and surfactant | | | | | | | |
|---|---|---|---|---|---|---|---|
| (The numerical values shown with the names of sodium polystyrene sulfonate, sodium polyacrylate, and polyacrylic acid show the weight average molecular weight of each compound) | | | | | | | |
| | | Relative ratio (%) | | | | | Determination |
| | | First time | Second time | Third time | Fourth time | Fifth time | |
| Comparative Example 13 | Not added (Control) | 125.7 | 117.5 | 121.2 | 115.4 | 114.2 | - |
| Example 8 | + 0.01% Sodium polystyrene sulfonate -70000 | 101.6 | 100.2 | 100.4 | 96.2 | 101.9 | A |
| Example 9 | + 0.01% Sodium polystyrene sulfonate -200000 | 100.5 | 101.8 | 97.0 | 96.1 | 98.5 | A |
| Example 10 | + 0.01% Sodium polystyrene sulfonate -1000000 | 99.1 | 99.1 | 95.5 | 89.4 | 97.8 | A |
| Example 11 | + 0.01% DEMOL RN | 104.9 | 99.2 | 103.5 | 101.8 | 103.7 | A |
| Example 12 | + 0.01% Sodium polyacrylate 22000-70000 | 113.9 | 108.2 | 103.9 | 103.3 | 107.0 | B |
| Example 13 | + 0.1% Polyacrylic acid 5000 | 109.9 | 101.9 | 100.5 | 92.7 | 98.6 | A |
| Example 14 | + 0.01% Polyacrylic acid 250000 | 117.1 | 105.8 | 105.8 | 102.9 | 104.5 | B |
| Example 15 | + 0.01% Polyacrylic acid 1000000 | 115.6 | 111.6 | 109.9 | 108.2 | 108.0 | C |
| Comparative Example 14 | + 0.01% Sodium cholate | 123.9 | 117.7 | 117.7 | 112.6 | 115.0 | D |
| Comparative Example 15 | + 0.01% SDS | 121.7 | 113.2 | 114.0 | 109.9 | 110.6 | D |
| Comparative Example 16 | + 0.01% ENAGICOL L-30AN | 123.1 | 116.6 | 118.5 | 114.0 | 110.0 | D |

Then, the results obtained when a high-molecular compound and a surfactant were added will be described (Table 4). Under the "Determination" column, "A" shows that the relative ratio of the first time at the second set was within 90 to 110%, thereby being a very good result, "B" shows that the relative ratio of the first time at the second set deviated from 90 to 110% but the relative ratio of the second time was within 90 to 110%, thereby being a good result, "C" shows that the relative ratios of the first and second times at the second set deviated from 90 to 110% but the third time was 90 to 110%, thereby being effective to some extent, and "D" shows that no effect was found.

When sodium polystyrene sulfonate, which is a high-molecular compound in which a monomer having a sulfo group is polymerized, (Example 8: Mw 70,000, Example 9: Mw 200,000, Example 10: Mw 1,000,000), and DEMOL RN (Example 11) were used, the relative ratio of the measurement sensitivity of the first time at the second set to the average measurement sensitivity of the five measurements at first set was 99.1 to 104.9%, reducing the carryover of sample B. The effect to some extent was also found with sodium polyacrylate (Example 12, molecular weight 22,000 to 70,000), and polyacrylic acid (Examples 13 to 15, molecular weight 5,000 to 1,000,000), high-molecular compounds in which a monomer having a carboxyl group is polymerized. Even further better results were expected to obtain when the molecular weight, concentration, and solution conditions are optimized. On the other hand, sodium cholate, sodium lauryl sulfate (SDS), and ENAGICOL L-30AN did not show the carryover reduction effect (Comparative Examples 14 to 16).

The above findings revealed that the diluents, when sodium polystyrene sulfonate (molecular weight 70,000 to 1,000,000), a naphthalene sulfonic acid condensate (DEMOL RN), sodium polyacrylate (molecular weight 22,000 to 70,000), and polyacrylic acid (molecular weight 5,000 to 1,000,000), which are acidic polymers, were added thereto, were capable of reducing the carryover caused when the TARC antigen adsorbed onto the sample probe. Accordingly, it was considered that when an acidic polymer was added to the diluent, the measurement of a sample having a low concentration of the TARC antigen is possible with high accuracy even when the sample having a low concentration of the TARC antigen is measured immediately after a sample having a high concentration of the TARC antigen is measured.

DEMOL RN also has a surfactant action but is considered as an acidic polymer in this description because it has sulfonic acid in a molecule.

**[Table 5]**

| Results when adding compounds having a positive charge in a molecule | | | | | | | |
|---|---|---|---|---|---|---|---|
| | | Relative ratio (%) | | | | | Determination |
| | | First time | Second time | Third time | Fourth time | Fifth time | |
| Comparative Example 1 | Not added (Control) | 178.5 | 151.4 | 141.2 | 135.1 | 120.2 | - |
| Comparative Example 17 | + 0.01% ACETAMIN 24P | 149.2 | 150.7 | 146.4 | 138.0 | 129.1 | D |
| Comparative Example 18 | + 0.05% ACETAMIN 24P | 192.5 | 165.1 | 148.4 | 143.9 | 134.9 | D |
| Comparative Example 19 | + 0.01% KOTAMIN 24P | 170.3 | 145.2 | 139.1 | 136.9 | 118.6 | D |
| Comparative Example 20 | + 0.05% KOTAMIN 24P | 169.2 | 155.1 | 143.8 | 139.2 | 131.3 | D |
| Comparative Example 21 | + 0.05% Lipidure-RL502 | 174.1 | 155.9 | 149.0 | 144.4 | 140.6 | D |
| Comparative Example 22 | + 0.1% Lipidure-BL502 | 170.7 | 151.0 | 145.6 | 138.0 | 139.7 | D |
| Comparative Example 23 | + 0.2% Lipidure-BL502 | 159.8 | 145.8 | 136.2 | 133.2 | 126.3 | D |
| Comparative Example 24 | + 0.1% Lipidure-NH01 | 152.1 | 153.6 | 143.4 | 134.6 | 134.4 | D |
| Comparative Example 25 | +10-Fold dilution Blockmaster CE210 | 183.6 | 153.2 | 140.5 | 139.8 | 134.6 | D |
| Comparative Example 26 | +10-Fold dilution Blockmaster CE510 | 167.9 | 153.2 | 120.0 | 137.1 | 126.6 | D |

Table 5 shows the study results on compounds having a positive charge in a molecule such as an amino group or a quaternary ammonium salt. The evaluation criteria are the same as Table 2 and Table 3, but none of them had the effect by the addition with respect to the not added (Comparative Example 1).

**[Table 6]**

| Study on addition concentration of acidic polymer | | | | | | | | |
|---|---|---|---|---|---|---|---|---|
| (The numerical values shown with the names of the compounds show the weight average molecular weight of each compound) | | | | | | | | |
| Number | Additive | Addition concentration | Relative ratio (%) | | | | | Determination |
| | | | First time | Second time | Third time | Fourth time | Fifth time | |
| Comparative Example 1 | Not added | | 155.5 | 138.1 | 125.3 | 119.6 | 111.8 | |
| Example 16 | Sodium polystyrene sulfonate -70,000 | 0.002% | 100.8 | 100.6 | 103.6 | 101.5 | 99.8 | A |
| Example 17 | | 0.005% | 100.6 | 104.1 | 105.9 | 99.4 | 99.0 | A |
| Example 18 | | 0.010% | 106.4 | 98.6 | 95.8 | 94.8 | 99.0 | A |
| Example 19 | | 0.020% | 102.4 | 103.0 | 102.0 | 96.7 | 96.9 | A |
| Example 20 | | 0.050% | 97.2 | 98.5 | 97.9 | 98.3 | 93.8 | A |
| Example 21 | Sodium polystyrene sulfonate -200,000 | 0.002% | 93.8 | 100.7 | 88.2 | 99.0 | 93.8 | A |
| Example 22 | | 0.005% | 100.7 | 99.4 | 100.5 | 99.2 | 100.7 | A |
| Example 23 | | 0.010% | 99.0 | 101.6 | 98.0 | 103.5 | 88.8 | A |
| Example 24 | | 0.020% | 104.7 | 96.1 | 104.3 | 99.8 | 102.1 | A |
| Example 25 | | 0.050% | 97.8 | 100.9 | 104.0 | 101.4 | 102.7 | A |
| Example 26 | Sodium polystyrene sulfonate - | 0.002% | 103.0 | 101.0 | 103.0 | 104.0 | 98.4 | A |
| Example 27 | | 0.005% | 102.3 | 100.0 | 97.5 | 96.7 | 100.0 | A |
| Example 28 | 1,000,000 | 0.010% | 96.6 | 94.9 | 96.2 | 96.8 | 98.4 | A |
| Example 29 | | 0.020% | 102.8 | 101.3 | 103.7 | 97.7 | 95.8 | A |
| Example 30 | | 0.050% | 101.0 | 98.1 | 99.0 | 95.6 | 99.2 | A |

Then, the results obtained when the addition concentration of sodium polystyrene sulfonate, which is an acidic polymer, was changed will be described (Table 6). Under the "Determination" column, "A" shows that the relative ratio of the first time at the second set was within 90 to 110%, thereby being a very good result.

When the addition concentration of sodium polystyrene sulfonate was changed from 0.002% to 0.050% (Examples 16 to 20: Mw 70,000, Examples 21 to 25: Mw 200,000, Examples 26 to 30: Mw 1,000,000), the relative ratio of the measurement sensitivity of the first time at the second set to the average measurement sensitivity of the five measurements at first set was 93.8 to 106.4%, reducing the carryover of sample B with the addition concentrations of sodium polystyrene sulfonate from 0.002% to 0.050%.

### Industrial Applicability

The present invention can provide a diluent for a calibration sample and a sample that is less likely to cause a carryover of the TARC antigen.

## Claims

1. A composition comprising:
(A) TARC (Thymus and activation-regulated chemokine); and
(B) one or more components selected from the group consisting of acidic amino acids, basic amino acids, acidic polymers having an average molecular weight of 4,000 to 1,200,000, and salts thereof, and
being in liquid form.

2. The composition according to claim 1, wherein the composition is a calibration sample solution for measuring TARC.

3. The composition according to claim 1 or 2, further comprising:
(C) a buffer.

4. The composition according to any one of claims 1 to 3, wherein
the acidic polymer and a salt thereof are sodium polystyrene sulfonate, a naphthalene sulfonic acid condensate, sodium polyacrylate, or polyacrylic acid.

5. The composition according to any one of claims 1 to 4, wherein a concentration of the component (B) is 0.0001 to 20 mass% with respect to the composition.

6. A diluent for a sample containing TARC, comprising:
(B) one or more components selected from the group consisting of acidic amino acids, basic amino acids, acidic polymers having an average molecular weight of 4,000 to 1,200,000, and salts thereof.

7. The diluent according to claim 6, wherein the acidic polymer and a salt thereof are sodium polystyrene sulfonate, a naphthalene sulfonic acid condensate, sodium polyacrylate, or polyacrylic acid.

8. The diluent according to claim 6 or 7, further comprising:
(C) a buffer.

9. The diluent according to any one of claims 6 to 8, wherein a concentration of the component (B) is 0.0001 to 20 mass% with respect to the diluent.

10. A method for reducing a TARC carryover, comprising: contacting:
(A) TARC with
(B) one or more components selected from the group consisting of acidic amino acids, basic amino acids, acidic polymers having an average molecular weight of 4,000 to 1,200,000, and salts thereof
in a solution.

11. The method for reducing a TARC carryover according to claim 10, wherein the acidic polymer and a salt thereof are sodium polystyrene sulfonate, a naphthalene sulfonic acid condensate, sodium polyacrylate, or polyacrylic acid.

12. The method for reducing a TARC carryover according to claim 10 or 11, wherein the solution is a buffer.

13. The method for reducing a TARC carryover according to any one of claims 10 to 12, wherein the carryover is a carryover in a sample probe.

14. The method for reducing a TARC carryover according to any one of claims 10 to 13, wherein a concentration of the component (B) is 0.0001 to 20 mass% with respect to the solution.

15. An adsorption inhibitor for preventing adsorption of TARC in a solution containing TARC onto a sample probe, comprising:
(B) one or more components selected from the group consisting of acidic amino acids, basic amino acids, acidic polymers having an average molecular weight of 4,000 to 1,200,000, and salts thereof.

16. The adsorption inhibitor according to claim 15, wherein the acidic polymer and a salt thereof are sodium polystyrene sulfonate, a naphthalene sulfonic acid condensate, sodium polyacrylate, or polyacrylic acid.

17. A method for continuously analyzing two or more samples containing TARC, the method comprising:
(a) suctioning a first sample containing TARC using a sample probe,
(b) discharging the first sample from the sample probe,
(c) measuring a signal intensity of the first sample,
(d) rinsing the wall surface of the sample probe with a rinsing solution,
(e) suctioning a second sample containing TARC using the sample probe, and
(f) measuring a signal intensity of the second sample,
wherein the first sample and the second sample comprise (B) one or more components selected from the group consisting of acidic amino acids, basic amino acids, acidic polymers having an average molecular weight of 4,000 to 1,200,000, and salts thereof, at 0.0001 mass% to 20 mass%, respectively, with respect to the first sample and the second sample, and
a TARC concentration of the first sample containing TARC is higher than a TARC concentration of the second sample containing TARC.
